# EUROPÄISCHE PATENTANMELDUNG

(11) **EP 4 181 041 A1**
(43) Veröffentlichungstag der Anmeldung: **17.05.2023**
(21) Anmeldenummer: 22207774.5
(22) Anmeldetag: 16.11.2022
(51) Int. Cl.: G06Q 10/0631, G16H 40/67, G16H 80/00, G16H 40/20

(54) **COMPUTERIMPLEMENTIERTES VERFAHREN ZUM MANAGEN EINES DIGITALEN INFORMATIONSAUSTAUSCHES IM ZUSAMMENHANG MIT RETTUNGSEINSÄTZEN UND VERFAHREN ZUR KOORDINATION VON IN EINEM NOTARZT-POOL VERFÜGBAREN NOTÄRZTEN**

(30) Priorität: 16.11.2021 DE 102021129885
(71) Anmelder: peiker Holding GmbH, 61352 Bad Homburg (DE)
(72) Erfinder: Timme, Sebastian, 12169 Berlin (DE); Fernholz, Erik, 12305 Berlin (DE)
(74) Vertreter: Otten, Roth, Dobler & Partner mbB Patentanwälte

(57) **Zusammenfassung**

Die Erfindung betrifft ein computerimplementiertes Verfahren zum Managen eines digitalen Informationsaustausches im Zusammenhang mit Rettungseinsätzen, wobei das Verfahren in ein elektronisches Organisationssystem (2) implementiert ist. Weiterhin betrifft die Erfindung ein Verfahren zur Koordination von in einem Notarzt-Pool verfügbaren Notärzten (501; 601).

## Beschreibung

Die Erfindung betrifft ein computerimplementiertes Verfahren zum Managen eines digitalen Informationsaustausches im Zusammenhang mit Rettungseinsätzen gemäß dem Oberbegriff des Anspruchs 1. Weiterhin betrifft die Erfindung ein computerimplementiertes Verfahren zur Koordination von in einem Notarzt-Pool verfügbaren Notärzten gemäß dem Anspruch 11.

Aus der DE 10 2020 109 040 A1 ist ein Verfahren zum Betrieb eines Telenotarztsystems bekannt, welches die nachfolgend genannten Schritte umfasst:
- Filmen eines Patienten mit einer von einem Nutzer getragenen tragbaren Digitalkamera;
- Generieren einer Kollektion wesentlicher Merkmale eines Patienten auf der Basis der durch das Filmen erfassten Digitalbilder, wobei die Kollektion wesentlicher Merkmale insbesondere aus einem Gesicht des Patienten generiert wird und wobei die Generierung der Kollektion durch die tragbare Digitalkamera oder durch wenigstens einen der Router oder durch den Sever erfolgt;
- Vergleichen der Kollektion der wesentlichen Merkmale des Patienten mit den laufend in dem Fahrzeug von einer dortigen Digitalkamera aufgenommenen Digitalbildern mittels eines Algorithmus, wobei der Algorithmus in die Digitalkameras des Fahrzeugs oder in den Router oder in den Server implementiert ist;
- Starten einer Übertragung der in dem Fahrzeug aufgenommenen Digitalbilder an einen Bildschirm eines Überwachungsmoduls, sobald das Vergleichen zu dem Ergebnis führt, dass der von der tragbaren Digitalkamera bereits erfasste Patient von der in dem Fahrzeug angeordneten Digitalkamera erfasst wird.

Es ist Aufgabe der Erfindung, ein computerimplementiertes Verfahren zum Managen eines digitalen Informationsaustausches im Zusammenhang mit Rettungseinsätzen sowie ein Verfahren zur Koordination von in einem Notarzt-Pool verfügbaren Notärzten vorzuschlagen, durch welches jeweils der verfügbare Notarzt bzw. die verfügbaren Notärzte effizienter eingesetzt werden können.

Diese Aufgabe wird durch die Merkmale des Anspruchs 1 bzw. des Anspruchs 11 gelöst. In den jeweiligen Unteransprüchen sind vorteilhafte und zweckmäßige Weiterbildungen angegeben.

Bei dem erfindungsgemäßen computerimplementierten Verfahren zum Managen eines digitalen Informationsaustausches im Zusammenhang mit Rettungseinsätzen, welches in ein elektronisches Organisationssystem implementiert ist,
- welches über eine Kommunikationsverbindung mit wenigstens einer Leitstelle verbunden wird,
- welches über eine Kommunikationsverbindung mit wenigstens einem Rettungswagen und wenigstens einem dem jeweiligen Rettungswagen zugeordneten Notfallsanitäter verbunden wird und
- welches über eine Kommunikationsverbindung mit wenigstens einem Notarzt, insbesondere unabhängig von dessen Standort und dessen Reisegeschwindigkeit verbunden wird,
- wobei dem elektronischen Organisationssystem zu jedem verbundenen Notarzt ein Einsatzstatus und eine Notarztzuordnung zu einem Indikationskatalog gemeldet werden,
- wobei über das elektronische Organisationssystem situationsabhängig Kommunikationsdaten, insbesondere als Audiodaten und/oder Videodaten und/oder Standortdaten und/oder Zustandsdaten und/oder Steuerdaten ausgetauscht werden.

Hierdurch ist es möglich, dass von einem Notarzt wenigstens zeitweise mehrere Rettungseinsätze betreut werden. Hierdurch ist es auch möglich, dass ein Notarzt mit Spezialkenntnissen zur Betreuung herangezogen wird. Weiterhin ist es hierdurch auch möglich, dass ein Notarzt, welcher sich auf der An- zu einem Rettungseinsatz oder auf der Abfahrt von einem Rettungseinsatz befindet zur wenigstens vorübergehenden Betreuung eines Rettungseinsatzes herangezogen wird.

Weiterhin ist es bei dem computerimplementierten Verfahren vorgesehen
- eine Notarztkonsultation durch den Notfallsanitäter über das elektronische Organisationssystem zu initiieren, wobei von dem elektronischen Organisationssystem auf der Basis von Geokoordinaten des an einem jeweiligen Unfallort befindlichen Rettungswagens und insbesondere auf der Basis einer Indikation automatisch zu demjenigen Notarzt eine Datenverbindung aufgebaut wird, welcher hinsichtlich der Kriterien Einsatzstatus und Notarztzuordnung und/oder hinsichtlich des Kriteriums kürzeste Wegzeit zum Unfallort als für eine Notarztkonsultation geeignet ausgewählt wird,
- zeitgleich zu der Initiierung der Notarztkonsultation die Leitstelle von dem elektronischen Organisationssystem über die Initiierung der Notarztkonsultation zu informieren und der Leitstelle von dem Organisationssystem teilweisen oder vollständigen Zugriff auf die zwischen dem Notfallsanitäter und dem ausgewählten Notarzt und/oder Facharzt geführte Kommunikation zu gewähren. Hierdurch ist automatisch eine zielgerichtete und schnelle Auswahl eines geeigneten Notarztes möglich, wobei die Leitstelle trotz des hohen Automatisierungsgrads ohne Zeitversatz informiert bleibt und somit in Sonderfällen auch eingreifen kann.

Es ist bei dem computerimplementierten Verfahren auch vorgesehen,
- eine Alarmierung des Rettungswagens durch die Leitstelle bei einem der Leitstelle gemeldeten Unfallereignis über das Organisationssystem durchzuführen,
- eine Bestätigung der Alarmierung durch den Notfallsanitäter an die Leitstelle über das Organisationssystem durchzuführen,
- und eine Bestätigung eines Eintreffens am Unfallort durch den Notfallsanitäter über das Organisationssystem durchzuführen. Hierdurch ist mit dem computerimplementierten Verfahren auch eine herkömmlich Alarmierung des Notarztes möglich. Dies erleichtert einen Wechsel auf das computerimplementierte Verfahren, da so eine schrittweise Modernisierung möglich ist.

Es ist auch vorgesehen, ein weiteres Einsatzkriterium für jeden Notarzt dadurch zu erstellen, dass von dem Organisationssystem automatisch ermittelt wird, ob der jeweiligen Notarzt von seinem Arbeitsplatz über einen ortsfesten Router oder von unterwegs mittels eines mobilen Routers mit dem elektronischen Organisationssystem verbunden ist. Hierdurch können von dem computerimplementierten Verfahren weitere Faktoren wie zum Beispiel maximal möglich Datenrate, vorübergehender Verbindungsausfall beachtet werden.

Es kann vorkommen, dass durch Störungen in der Netzversorgung die Datenrate reduziert wird. Bei reduzierter, das heißt nicht für die Übertragung aller zur Übertragung bereitstehender Daten ausreichender Datenrate, wird von dem Organisationssystem eine Priorisierung für die Datenübertragung vorgegeben, wobei es insbesondere vorgesehen ist, dass die Sprachkommunikation die höchste Priorität erhält und dass die Übertragung der Vitaldaten des Patienten die zweithöchste Priorität erhält, wobei die Übertragung eines Live-Video vorzugsweise die niedrigste Priorität erhält. Hierbei kann das computerimplementierte Verfahren auch vorsehen, dass die Priorisierung von dem Notarzt modifiziert wird. Hierdurch lässt sich die Priorisierung optimal auf das individuelle Einsatzszenario anpassen.

Weiterhin ist es vorgesehen, dass dem Organisationssystem von dem Notarzt oder dem Facharzt, insbesondere mittels Spracheingabe oder Menüauswahl Befehle zur Ausführung von programmierbaren Routinen gegeben werden können, durch welche Rettungskräfte und/oder Hilfskräfte und/oder weitere Fachkräfte automatisch in das Organisationssystem eingebunden werden und mit den verfügbaren Daten versorgt werden, wobei als Fachkräfte insbesondere Fachärzte eingebunden werden. Hierdurch lässt sich im Rettungsablauf insbesondere ein Zeitgewinn realisieren. Weiterhin ermöglicht es ein derartiger Automatisierungsgrad, dass der Notarzt und/oder der Facharzt nur minimal durch administrative Tätigkeiten von seinen medizinischen Aufgaben abgelenkt ist bzw. sind.

Es ist auch vorgesehen, das elektronische Organisationssystem mit einer zentralen Recheneinheit auszustatten und das elektronische Organisationssystem mit Endgeräten auszustatten, welche an dieses jeweils über wenigstens einen bidirektionalen Kommunikationsweg angebunden sind. Hierdurch kann der Gesamtablauf zentral gesteuert werden.

Weiterhin ist es bei dem computerimplementierten Verfahren vorgesehen,
- die Koordinaten eines Aufenthaltsort des Notarztes als Steuerdaten für das Einsatzkriterium "Wegdauer zum Unfallort" anzusehen zu verarbeiten und/oder
- eine Anzahl von laufenden Konsultationen, in welche der Notarzt involviert ist, als Steuerdaten für das Einsatzkriterium "Auslastung des Notarztes" zu verarbeiten und/oder
- eine Bezeichnung des medizinischen Spezialgebiets des Notarztes als Steuerdaten für das Einsatzkriterium "Spezialisierung" zu verarbeiten. Aus einem Abgleich derartige Steuerdaten lässt sich der geeignetste Notarzt aus einer Vielzahl von zur Verfügung stehenden Notärzten einfach und schnell ermitteln. Hierdurch lassen sich auch restriktive Vorgaben aus einem vorgegebenen Alarmierungskatalog für den Telenotarzt zur Auswahl des jeweils geeigneten Notarztes verwerten.

Es ist auch vorgesehen, einen stationären Arbeitsplatz des Notarztes und/oder einen mobilen Arbeitsplatz des Notarztes derart auszubilden, dass dieser Multisession-fähig ist, so dass für den Notarzt bei Bedarf parallele Verbindungen zu mehreren Unfallorten hergestellt werden. Hierdurch kann auch in Falle einer Unterversorgung mit Notärzten an jedem Unfallort eine Notversorgung sichergestellt werden.

Weiterhin kann eine Priorisierung der Übertragung der verschiedenen Datenarten wie insbesondere der Datenarten Sprachdaten und Vitaldaten und Bilddaten und Life-Videodaten derart vorgenommen werden, dass abhängig von einer möglichen Übertragungsqualität und/oder Übertragungsrate oder abhängig von z.B. auf einer auf einer Wegstrecke z.B. einer Fahrt vom Unfallort zum Krankenhaus zu erwartenden Übertragungsqualität und/oder Übertragungsrate eine automatisierte oder von dem Notfallsanitäter oder von dem Notarzt oder von der Leitstelle sowohl aktivierbare, als auch deaktivierbare Priorisierung der Datenarten erfolgt und die Datenarten insbesondere in einer Priorisierungsreihenfolge Sprachdaten, Vitaldaten des Unfallopfers, Bilddaten und Life-Videodaten für die Übertragung priorisiert werden. Hierdurch kam trotz schwieriger Bedingungen eine Grundversorgung durch den Notarzt sichergestellt werden.

Es kann auch vorgesehen sein, die Priorisierungsreihenfolge letztlich von dem Notarzt zu bestimmen. Hierdurch kann dieser abhängig von den medizinischen Erfordernissen z.B. zwischen einer Übertragung von Sprachdaten und Vitaldaten des Unfallopfers wechseln oder z.B. kurzfristig auch die ausschließliche Übertragung von Life-Videodaten bevorzugen, wenn dies z.B. zur Diagnose erforderlich ist.

Bei dem erfindungsgemäßen Verfahren zur Koordination von in einem Notarzt-Pool verfügbaren Notärzten,
- umfasst der Notarzt-Pool wenigstens einen "mobilen Notarzt", dessen Notarztwagen derart mit Kommunikationsmitteln ausgestattet ist, dass sich der Notarzt auf der Anfahrt dem bereits an einem Unfallort befindlichen Notfallsanitäter zuschalten kann,
- umfasst der Notarzt-Pool wenigstens einen "Telenotarzt", dessen Stützpunkt derart ausgestattet ist, dass sich der Notarzt dem bereits am Unfallort befindlichen Notfallsanitäter zuschalten kann,
- wobei jedem Notarzt
   o ein erster Kennwert zugeordnet ist, durch welchen dessen Notarztstatus, nämlich "mobiler Notarzt" oder "Telenotarzt" angegeben ist,
   o ein zweiter Kennwert zugeordnet ist, durch welchen dessen Einsatzstatus, nämlich "verfügbar" oder "nicht verfügbar" angegeben ist,
   o ein dritter Kennwert zugeordnet ist, durch welchen angegeben ist, für welche Indikationen aus einem Indikationskatalog der Notarzt Spezialist ist,
- wobei von einem elektronischen Organisationssystem auf der Basis der Kennwerte, auf der Basis einer Unfallmeldung und auf der Basis von durch den Notfallsanitäter gemachten Angaben eine Auswahl erfolgt,
   o welche entweder der Leitstelle als Vorschlag für einen zu beauftragenden Notarzt aus dem Notarzt-Pool übermittelt wird
   o oder durch welche ein Notarzt aus dem Notarzt-Pool automatisch beauftragt wird.

Hierdurch ist es auch möglich, dass ein Notarzt mit Spezialkenntnissen zur Betreuung herangezogen wird. Weiterhin ist es hierdurch auch möglich, dass ein Notarzt, welcher sich auf der An- zu einem Rettungseinsatz oder auf der Abfahrt von einem Rettungseinsatz befindet zur wenigstens vorübergehenden Betreuung eines Rettungseinsatzes herangezogen wird.

Es ist auch vorgesehen, sofern ein "mobiler Notarzt" zur Beauftragung ausgewählt wird, bei allen "mobilen Notärzten" als vierten Kennwert den Standort zu erfassen und die Auswahl des Notarztes auch von einer errechneten Wegzeit zu einem Unfallort abhängig zu machen.

Weiterhin ist es vorgesehen, als fünften Kennwert ein den "mobilen Notärzten" jeweils zur Verfügung stehendes Verkehrsmittel, welches insbesondere als Straßenfahrzeug oder Wasserfahrzeug oder Luftfahrzeug ausgebildet sein kann, zu erfassen. Hierdurch wird eine automatische Auswahl eines geeigneten Notarztes weiter verbessert.

Es ist auch vorgesehen, einen stationären Arbeitsplatz des Notarztes und/oder einen mobilen Arbeitsplatz des Notarztes derart auszubilden, dass dieser Multisession-fähig ist, so dass für den Notarzt bei Bedarf parallele Verbindungen zu mehreren Unfallorten hergestellt werden. Hierdurch kann auch in Falle einer Unterversorgung mit Notärzten an jedem Unfallort eine Notversorgung sichergestellt werden. Entsprechend ist es auch vorgesehen, dass bei einer parallelen Betreuung unterschiedlicher Notfälle durch den selben Notarzt, eine Bildschirmdarstellung jedes Notfalls automatisch oder vom Notarzt selbst angepasst wird, so dass der Notfallsituation entsprechend zu jedem Notfall Daten in unterschiedlichem Umfang angezeigt werden. Hierdurch ist es möglich, die Informationsmenge ohne Qualitätseinbußen zu reduzieren, wobei z.B. bei einem Notfall, welcher einen unkomplizierten Krankentransport umfasst, nur die Vitaldaten des Patienten angezeigt werden.

Schließlich ist es auch vorgesehen, den zweiten Kennwert aus einem elektronischen Dienstplan zu ermitteln oder den zweiten Kennwert mittels eines elektronischen Dienstplans zu verifizieren. Entsprechend der ersten Variante lässt sich der zweite Kennwert auf einfache Weise aus einem bestehenden Planungssystem entnehmen. Entsprechend der zweite Variante, das heißt, wenn der Einsatzstatus z.B. anhand einer automatischen Statuserkennung oder anhand eines von Notarzt bzw. Telenotarzt selbst gesetzten Status erfolgt, lässt sich die Plausibilität des vorliegenden Status einfach überprüfen, somit können Fehlinformationen automatisch erkannt werden.

Im Sinne der Erfindung wird unter einem Managen eines digitalen Informationsaustausches das Steuern und Kontrollieren dieses digitalen Informationsaustausches verstanden.

Weitere Einzelheiten der Erfindung werden in der Zeichnung anhand von schematisch dargestellten Ausführungsbeispielen beschrieben.

Hierbei zeigt:

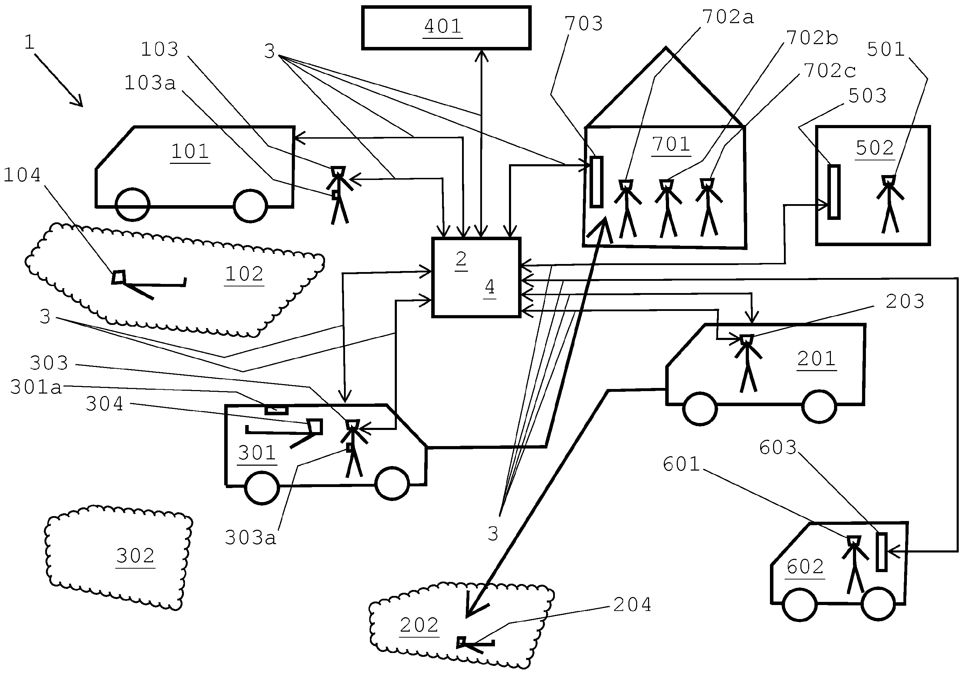

- Figur 1:: eine schematische Darstellung eines Einsatzszenarios, an welchem das erfindungsgemäße computerimplementierte Verfahren zum Managen eines digitalen Informationsaustausches im Zusammenhang mit Rettungseinsätzen erläutert ist und an welchem das erfindungsgemäße Verfahren zur Koordination von in einem Notarzt-Pool verfügbaren Notärzten erläutert ist.

In der Figur 1 ist schematisch ein Einsatzszenario 1 dargestellt, an welchem das erfindungsgemäße computerimplementierte Verfahren zum Managen eines digitalen Informationsaustausches im Zusammenhang mit Rettungseinsätzen erläutert ist und an welchem das erfindungsgemäße Verfahren zur Koordination von in einem Notarzt-Pool verfügbaren Notärzten erläutert ist.

Das Einsatzszenario 1 zeigt einen ersten Rettungswagen 101 einer Vielzahl von Rettungswägen, welcher sich an einem ersten Unfallort 102 befindet. Dem Rettungswagen 101 ist ein erster Notfallsanitäter 103 zugeordnet, welcher sich außerhalb des Rettungswagens 101 um ein erstes Unfallopfer 104 kümmert.

Das Einsatzszenario 1 zeigt einen zweiten Rettungswagen 201 der Vielzahl von Rettungswägen, welcher sich auf dem Weg zu einem zweiten Unfallort 202 befindet. Dem zweiten Rettungswagen 201 ist ein zweiter Notfallsanitäter 203 zugeordnet. An dem Unfallort 202 befindet sich ein zweites Unfallopfer 204.

Das Einsatzszenario 1 zeigt einen dritten Rettungswagen 301 der Vielzahl von Rettungswägen, welcher sich auf dem Rückweg von einem dritten Unfallort 302 befindet. Dem dritten Rettungswagen 301 ist ein dritter Notfallsanitäter 303 zugeordnet, welcher sich in dem dritten Rettungswagen 301 um ein drittes Unfallopfer 304 kümmert.

Das Einsatzszenario 1 zeigt auch eine Leitstelle 401.

Weiterhin zeigt das Einsatzszenario 1 einen ersten Notarzt 501, welcher stationär an einem ersten Standort 502 für Einsätze zur Verfügung steht und einen zweiten Notarzt 601, welcher mobil in einem Notarztwagen 602 unterwegs ist und für Einsätze zur Verfügung steht. Der erste Notarzt 501 wird auch als Telenotarzt oder stationärer Notarzt bezeichnet und der zweite Notarzt 601 wird auch als mobiler Notarzt bezeichnet.

Das Einsatzszenario 1 zeigt auch ein Krankenhaus 701, in welchem eine Vielzahl von Fachärzten 702a, 702b und 702c Dienst haben.

Schließlich zeigt das Einsatzszenario 1 schematisch ein elektronisches Organisationssystem 2, in welches das Verfahren zum Managen eines digitalen Informationsaustausches im Zusammenhang mit Rettungseinsätzen implementiert ist.

Das elektronische Organisationssystem 2 ist über Kommunikationsverbindungen 3 mit der Leitstelle 401, mit dem ersten Rettungswagen 101 und dem ersten Notfallsanitäter 103, mit dem zweiten Rettungswagen 201 und dem zweiten Notfallsanitäter 203, mit dem dritten Rettungswagen 301 und dem dritten Notfallsanitäter 303, mit dem ersten Notarzt 501 über ein Terminal 503, mit dem zweiten Notarzt 601 über ein Terminal 603 und mit den Fachärzten 702a-702c über ein Terminal 703 verbunden.

Dem elektronischen Organisationssystem 2 sind zu jedem der verbundenen Notärzte 501, 601 ein Einsatzstatus und eine Notarztzuordnung zu einem Indikationskatalog gemeldet.

Weiterhin werden über das elektronische Organisationssystem 2 situationsabhängig Kommunikationsdaten, als Audiodaten und Videodaten und Standortdaten und Zustandsdaten und Steuerdaten ausgetauscht.

Hierbei erfolgt in dem dargestellten Einsatzszenario 1 über das elektronische Organisationssystem 2 zwischen dem ersten Notfallsanitäter 103 und dem ersten Notarzt 501 ein Austausch von Audiodaten, Videodaten und Zustandsdaten. Hierdurch kann der erste Notarzt 501 mittels einer von dem ersten Notfallsanitäter 103 getragenen Bodycam 103a das erste Unfallopfer 104 beobachten, steht gleichzeitig in Sprachverbindung mit dem ersten Notfallsanitäter 103 und bekommt als Zustandsdaten Vitaldaten des ersten Unfallopfers 102 übermittelt. Hierzu kommt z.B. ein nicht dargestelltes EKG zum Einsatz, welches ebenfalls drahtlos an das elektronische Organisationssystem angeschlossen ist.

Hierbei hat der zweite Rettungswagen 201 über das elektronische Organisationssystem 2 auf der Basis eines in der Leitstelle 401 eingegangenen Notrufs Geokoordinaten des zweiten Unfallorts 202 erhalten und ist auf dem Weg zu dem zweiten Unfallopfer 204. Weiterhin wurde von dem elektronischen Organisationssystem 2 auf Grund der Leitstelle 401 zu dem zweiten Unfallopfer 204 gemeldeten Daten "lebensgefährlichen Verletzung" und "Kleinkind" der zweite, mobile Notarzt 601 alarmiert, da dieser den zweiten Unfallort 202 am schnellsten erreichen kann und da die vermutete Unfallschwere einen Notarzt vor Ort erfordert. Hierbei wurde die für die Anfahrt zum zweiten Unfallort 202 erforderliche Zeit von dem elektronischen Organisationssystem 2 auf der Basis der Geokoordinaten des zweiten Unfallorts 202 und auf der Basis der Geokoordinaten, welche der Notarztwagen 602 des zweiten Notarztes 601 zum Zeitpunkt des Notrufs hatte, ermittelt. Ergänzend wurde von dem elektronischen Organisationssystem 2 aufgrund der zum Unfallopfer gemeldeten Daten in dem Krankenhaus 701 bereits einer der Fachärzte 702a-702c alarmiert, welcher über das in dem Krankenhaus 701 vorhandene Terminal 703 den zweiten Notarzt 602 unterstützen kann. Der Notarzt 601 kann sich dann dem Notfallsanitäter 203, welcher im vorliegenden Szenario vor dem Notarzt 601 an dem Unfallort 202 eintreffen wird, zuschalten und diesen bei den ersten Rettungsmaßnahmen anleiten. Hierzu nutzt der Notarzt 601 das Terminal 603.

Eine Versorgung des dritten Unfallopfers 304, welches sich in dem dritten Rettungswagen 301 auf dem Weg zu dem Krankenhaus 701 befindet, wird per Multisession durch den ersten Notarzt 501 unterstützt, welcher mittels einer in dem dritten Rettungswagen 301 vorhandenen Kamera 301a oder mittels einer von dem dritten Notfallsanitäter 303 getragenen Bodycam 303a das dritte Unfallopfer 304 beobachten kann, in Sprachverbindung mit dem dritten Notfallsanitäter 303 steht und als Zustandsdaten Vitaldaten des dritten Unfallopfers 304 übermittelt bekommt. Hierzu ist an dem ersten Standort 502 das Multisession-geeignete Terminal 503 vorhanden

Das elektronische Organisationssystem 2 bietet der Leitstelle 401 die Möglichkeit die Kommunikationsdaten der einzelnen Rettungseinsätze zu verfolgen, so dass z.B. bei einer Überlastung des Rettungssystem seitens der Leitstelle 401 die laufenden Rettungseinsätze verfolgt und eingestuft werden können und koordinierend eingegriffen werden kann.

Sowohl die Notfallsanitäter 103, 203, 303 als auch die Notärzte 501, 601, als auch die Rettungswägen 101, 201, 301, als auch der Notarztwagen 602 sind mit Endgeräten ausgestattet, welche die beschriebene Kommunikation innerhalb des elektronischen Organisationssystems ermöglichen.

Das elektronische Organisationssystem 2 umfasst eine zentrale Recheneinheit 4. Diese ist über die Kommunikationsverbindungen 3, welches als drahtlose, bidirektionale Kommunikationswege ausgebildet sind, an die verschiedenen Endgeräte wie z.B. die Bodycams 103a, 303a, die Kamera 301a und die Terminals 503, 603, 703 angebunden.

Ein Notarzt-Pool umfasst die Notärzte 501 und 601 sowie weitere nicht explizit genannten Notärzte, welche an Landkreisgrenzen auch mobile Notärzte aus anderen Landkreisen sein können und welche auch stationäre Notärzte aus beliebigen anderen Landkreisen sein können.

Es kann auch vorgesehen sein, dass es bei dem computerimplementierten Verfahren zum Managen eines digitalen Informationsaustausches im Zusammenhang mit Rettungseinsätzen vorgesehen ist, eine Priorisierung der Übertragung der verschiedenen Datenarten wie insbesondere der Datenarten Sprachdaten und Vitaldaten und Bilddaten und Life-Videodaten derart vorzunehmen, dass abhängig von einer möglichen Übertragungsqualität und/oder Übertragungsrate oder abhängig von z.B. auf einer auf einer Wegstrecke z.B. Fahrt vom Unfallort zum Krankenhaus zu erwartenden Übertragungsqualität und/oder Übertragungsrate eine automatisierte oder vom Notfallsanitäter oder vom Notarzt oder von der Leitstelle sowohl aktivierbare als auch deaktivierbare Priorisierung derart ausgeführt wird, dass eine Priorisierung der Datenarten erfolgt und eine Priorisierung insbesondere derart erfolgt, dass eine Priorisierungsreihenfolge Sprachdaten, Vitaldaten des Unfallopfers, Bilddaten und Life-Videodaten ausgewählt wird.

Bei dem computerimplementierten Verfahren ist es vorgesehen, dass die Priorisierungsreihenfolge letztlich von dem Notarzt bestimmt wird. Hierdurch kann dieser bei Bedarf bei niedriger Übertragungsqualität und/oder Übertragungsrate auch zwischen den Datenarten wechseln, um dem Notfallsanitäters eine optimale Unterstützung zu geben.

Hierdurch ist es auch möglich, dass der Notarzt einen oder mehrere Krankentransporte virtuell begleitet. Sofern das Terminal des Notarztes im Multisession-Modus betrieben wird, kann dieser auch mehrere Kranktransporte virtuell begleiten und die Priorisierung der Datenraten beispielsweise vom Zustand der einzelnen Patienten abhängig machen, wobei in der Regel eine Anzeige der Vitaldaten der einzelnen Patienten ausreichend sein wird.

### Bezugszeichenliste:

- 1: Einsatzszenario
- 2: elektronisches Organisationssystem
- 3: Kommunikationsverbindung zu 2
- 4: Recheneinheit

- 101: erster Rettungswagen
- 102: erster Unfallort
- 103: erster Notfallsanitäter
- 103a: Bodycam von 103
- 104: erstes Unfallopfer

- 201: zweiter Rettungswagen
- 202: zweiter Unfallort
- 203: zweiter Notfallsanitäter
- 204: zweites Unfallopfer

- 301: dritter Rettungswagen
- 301a: Kamera in 301
- 302: dritter Unfallort
- 303: dritter Notfallsanitäter
- 303a: Bodycam von 303
- 304: drittes Unfallopfer

- 401: Leitstelle

- 501: erster Notarzt bzw. Telenotarzt bzw. stationärer Notarzt
- 502: erster Standort
- 503: Multisession-geeignetes Terminal
- 601: zweiter Notarzt bzw. mobiler Notarzt
- 602: Notarztwagen von 601

- 701: Krankenhaus
- 702a-702c: Facharzt
- 703: Terminal

## Patentansprüche

1. Computerimplementiertes Verfahren zum Managen eines digitalen Informationsaustausches im Zusammenhang mit Rettungseinsätzen, wobei das Verfahren in ein elektronisches Organisationssystem (2) implementiert ist,
- welches über eine Kommunikationsverbindung mit wenigstens einer Leitstelle (401) verbunden wird,
- welches über eine Kommunikationsverbindung mit wenigstens einem Rettungswagen (101; 201; 301) und wenigstens einem dem jeweiligen Rettungswagen (101; 201; 301) zugeordneten Notfallsanitäter (103; 203; 303) verbunden wird und
- welches über eine Kommunikationsverbindung mit wenigstens einem Notarzt (501; 601) verbunden wird,
- wobei dem elektronischen Organisationssystem (2) zu jedem verbundenen Notarzt (501; 601) ein Einsatzstatus und eine Notarztzuordnung zu einem Indikationskatalog gemeldet werden,
- wobei über das elektronische Organisationssystem (2) situationsabhängig Kommunikationsdaten, insbesondere als Audiodaten und/oder Videodaten und/oder Standortdaten und/oder Zustandsdaten und/oder Steuerdaten ausgetauscht werden.

2. Computerimplementiertes Verfahren nach Anspruch 1, **dadurch gekennzeichnet,**
- **dass** eine Notarztkonsultation durch den Notfallsanitäter (103; 203; 303) über das elektronische Organisationssystem (2) initiiert wird, wobei von dem elektronischen Organisationssystem (2) auf der Basis von Geokoordinaten des an einem jeweiligen Unfallort (102; 202; 302) befindlichen Rettungswagens (101; 201; 301) und insbesondere auf der Basis einer Indikation automatisch zu demjenigen Notarzt (501; 601) eine Datenverbindung aufgebaut wird, welcher hinsichtlich der Kriterien Einsatzstatus und Notarztzuordnung und/oder hinsichtlich des Kriteriums kürzeste Wegzeit zum Unfallort (102; 202; 302) als für eine Notarztkonsultation geeignet ausgewählt wird,
- **dass** zeitgleich zu der Initiierung der Notarztkonsultation die Leitstelle (401) von dem elektronischen Organisationssystem (2) über die Initiierung der Notarztkonsultation informiert wird und dass der Leitstelle (401) von dem Organisationssystem 82) teilweiser oder vollständiger Zugriff auf die zwischen dem Notfallsanitäter (103; 203; 303) und dem ausgewählten Notarzt (501; 601) und/oder Facharzt (702a-702c) geführte Kommunikation gewährt wird.

3. Computerimplementiertes Verfahren nach wenigstens einem der vorhergehenden Ansprüche, **dadurch gekennzeichnet,**
- **dass** eine Alarmierung des Rettungswagens (101; 201; 301) durch die Leitstelle (401) bei einem der Leitstelle (401) gemeldeten Unfallereignis über das Organisationssystem (2) durchgeführt wird,
- **dass** eine Bestätigung der Alarmierung durch den Notfallsanitäter (103; 203; 303) an die Leitstelle (401) über das Organisationssystem (2) durchgeführt wird,
- **dass** eine Bestätigung eines Eintreffens am Unfallort (102; 202; 302) durch den Notfallsanitäter (103; 203; 303) über das Organisationssystem (2) durchgeführt wird.

4. Computerimplementiertes Verfahren nach wenigstens einem der vorhergehenden Ansprüche, **dadurch gekennzeichnet, dass** ein weiteres Einsatzkriterium für jeden Notarzt (501; 601) dadurch erstellt wird, dass von dem Organisationssystem (2) automatisch ermittelt wird, ob der jeweilige Notarzt (501; 601) von seinem Arbeitsplatz über einen ortsfesten Router oder von unterwegs mittels eines mobilen Routers mit dem elektronischen Organisationssystem (2) verbunden ist.

5. Computerimplementiertes Verfahren nach wenigstens einem der vorhergehenden Ansprüche, **dadurch gekennzeichnet, dass** dem Organisationssystem (2) von dem Notarzt (501; 601) oder von dem Facharzt (702a-702c), insbesondere mittels Spracheingabe oder Menüauswahl Befehle zur Ausführung von programmierbaren Routinen gegeben werden können, durch welche Rettungskräfte und/oder Hilfskräfte und/oder weitere Fachkräfte automatisch in das Organisationssystem eingebunden werden und mit den verfügbaren Daten versorgt werden, wobei als Fachkräfte insbesondere Fachärzte (702a-702c) eingebunden werden.

6. Computerimplementiertes Verfahren nach wenigstens einem der vorhergehenden Ansprüche, **dadurch gekennzeichnet, dass** das elektronische Organisationssystem (2) eine zentrale Recheneinheit (4) umfasst und dass das elektronische Organisationssystem (2) an diese über jeweils wenigstens einen bidirektionale Kommunikationsweg angebundene Endgeräte umfasst.

7. Computerimplementiertes Verfahren nach wenigstens einem der vorhergehenden Ansprüche, **dadurch gekennzeichnet,**
- **dass** Koordinaten eines Aufenthaltsort des Notarztes (501; 601) als Steuerdaten für das Einsatzkriterium "Wegdauer zum Unfallort" verarbeitet werden und/oder
- **dass** eine Anzahl von laufenden Konsultationen, in welche der Notarzt (501; 601) involviert ist, als Steuerdaten für das Einsatzkriterium "Auslastung des Notarztes" verarbeitet wird und/oder
- **dass** eine Bezeichnung des medizinischen Spezialgebiets des Notarztes (501; 601) als Steuerdaten für das Einsatzkriterium "Spezialisierung" verarbeitet wird.

8. Computerimplementiertes Verfahren nach wenigstens einem der vorhergehenden Ansprüche, **dadurch gekennzeichnet, dass** ein stationärer Arbeitsplatz des Notarztes (501) und/oder ein mobiler Arbeitsplatz des Notarztes (601) derart ausgebildet wird, dass dieser Multisession-fähig ist, so dass für den Notarzt (501; 601) bei Bedarf parallele Verbindungen zu mehreren Unfallorten (102; 202; 302) hergestellt werden.

9. Computerimplementiertes Verfahren nach wenigstens einem der vorhergehenden Ansprüche, **dadurch gekennzeichnet, dass** eine Priorisierung der Übertragung der verschiedenen Datenarten wie insbesondere der Datenarten Sprachdaten und Vitaldaten und Bilddaten und Life-Videodaten derart vorgenommen wird, dass abhängig von einer möglichen Übertragungsqualität und/oder Übertragungsrate oder abhängig von z.B. auf einer auf einer Wegstrecke z.B. einer Fahrt vom Unfallort zum Krankenhaus zu erwartenden Übertragungsqualität und/oder Übertragungsrate eine automatisierte oder von dem Notfallsanitäter oder von dem Notarzt oder von der Leitstelle sowohl aktivierbare, als auch deaktivierbare Priorisierung der Datenarten erfolgt und die Datenarten insbesondere in einer Priorisierungsreihenfolge Sprachdaten, Vitaldaten des Unfallopfers, Bilddaten und Life-Videodaten für die Übertragung priorisiert werden.

10. Computerimplementiertes Verfahren nach Anspruch 9, **dadurch gekennzeichnet, dass** die Priorisierungsreihenfolge letztlich von dem Notarzt bestimmt wird.

11. Verfahren zur Koordination von in einem Notarzt-Pool verfügbaren Notärzten,
- wobei der Notarzt-Pool wenigstens einen mobilen Notarzt (601) umfasst, dessen Notarztwagen (602) derart mit Kommunikationsmitteln ausgestattet ist, dass sich der Notarzt auf der Anfahrt dem bereits an einem Unfallort (202) befindlichen Notfallsanitäter zuschalten kann,
- wobei der Notarzt-Pool wenigstens einen sogenannten "Telenotarzt" (501) umfasst, dessen Stützpunkt derart ausgestattet ist, dass sich der Notarzt dem bereits am Unfallort befindlichen Notfallsanitäter (203) zuschalten kann,
- wobei jedem Notarzt (501; 601)
o ein erster Kennwert zugeordnet ist, durch welchen dessen Notarztstatus, nämlich "mobiler Notarzt" oder "Telenotarzt" angegeben ist,
o ein zweiter Kennwert zugeordnet ist, durch welchen dessen Einsatzstatus, nämlich "verfügbar" oder "nicht verfügbar" angegeben ist,
o ein dritter Kennwert zugeordnet ist, durch welchen angegeben ist, für welche Indikationen aus einem Indikationskatalog der Notarzt Spezialist ist,
- wobei von einem elektronischen Organisationssystem (2) auf der Basis der Kennwerte, auf der Basis einer Unfallmeldung und auf der Basis von durch den Notfallsanitäter (103; 203; 303) gemachten Angaben eine Auswahl erfolgt,
o welche entweder der Leitstelle (401) als Vorschlag für einen zu beauftragenden Notarzt (501; 601) aus dem Notarzt-Pool übermittelt wird
o oder durch welche ein Notarzt (501; 601) aus dem Notarzt-Pool automatisch beauftragt wird.

12. Verfahren nach Anspruch 11, **dadurch gekennzeichnet, dass** sofern ein "mobiler Notarzt" (601) zur Beauftragung ausgewählt wird, bei allen "mobilen Notärzten" (601) als vierter Kennwert der Standort erfasst wird und die Auswahl des Notarztes (601) durch eine errechnete Wegzeit zu einem Unfallort (102; 202; 302) beeinflusst wird.

13. Verfahren nach Anspruch 12, **dadurch gekennzeichnet, dass** als fünfter Kennwert ein den "mobilen Notärzten" (601) jeweils zur Verfügung stehendes Verkehrsmittel, welches insbesondere als Straßenfahrzeug oder Wasserfahrzeug oder Luftfahrzeug ausgebildet ist, erfasst wird.

14. Verfahren nach wenigstens einem der vorhergehenden Ansprüche 11 bis 13, **dadurch gekennzeichnet, dass** ein stationärer Arbeitsplatz des Notarztes (501) und/oder ein mobiler Arbeitsplatz des Notarztes (601) derart ausgebildet wird, dass dieser Multisession-fähig ist, so dass für den Notarzt (501; 601) bei Bedarf parallele Verbindungen zu mehreren Unfallorten (102; 202; 302) hergestellt werden.

15. Verfahren nach wenigstens einem der vorhergehenden Ansprüche 11, **dadurch gekennzeichnet, dass** der zweite Kennwert aus einem elektronischen Dienstplan ermittelt wird oder mittels eines elektronischen Dienstplans verifiziert wird.
